# EUROPÄISCHE PATENTSCHRIFT

(11) **EP 0 484 486 B1**
(45) Veröffentlichungstag und Bekanntmachung des Hinweises auf die Patenterteilung: **24.01.1996**
(21) Anmeldenummer: 91909332.8
(22) Anmeldetag: 21.05.1991
(51) Int. Cl.: A23L 1/03, A23L 1/30, A23L 2/52, A61K 31/20, A61K 31/23

(54) **VERFAHREN ZUR HERSTELLUNG EINES FRUCHTSAFT-GETRÄNKES**
PROCESS FOR PREPARING A FRUITJUICE DRINK
PROCEDE DE PREPARATION D'UNE BOISSON A BASE DE JUS DE FRUITS

(30) Priorität: 22.05.1990 CH 1745/90; 25.05.1990 CH 1784/90; 25.05.1990 CH 1783/90
(43) Veröffentlichungstag der Anmeldung: 13.05.1992
(73) Patentinhaber: BUMANN, Harold, CH-3906 Saas-Fee (CH)
(72) Erfinder: BUMANN, Harold, CH-3906 Saas-Fee (CH)
(74) Vertreter: Schmauder, Klaus Dieter
(86) Internationale Anmeldenummer: CH9100120
(87) Internationale Veröffentlichungsnummer: WO9117670

(56) Entgegenhaltungen:
- EP-A- 0 108 594
- WO-A-89/05101
- DE-A- 3 213 744
- GB-A- 2 140 806
- GB-A- 2 209 936
- US-A- 4 737 367

## Beschreibung

Die Erfindung betrifft ein Verfahren zu Herstellung eines Fruchtsaft-Getränkes gemäß Oberbegriff des Anspruches 1 sowie Anwendungen des Verfahrens gemäß den Ansprüchen 4 und 5.

Bekannt sind Lebensmittel mit bei fortgesetzter Einnahme prophylaktischer und/oder heilender Wirkung zur Verhütung und/oder Heilung bestimmter Krankheiten, hauptsächlich Zivilisationskrankheiten, die durch ungesunde Ernährungsweise, Alkohol- und Nikotinmissbrauch und durch eine zunehmende Umweltbelastung bedingt sind, wie beispielsweise cardiovaskuläre Erkrankungen, z.B. Herzinfarkt und Arteriosklerose, sowie Krebs. Das Lebensmittel liegt vorzugsweise in Form eines Getränkes, z.B. auf der Basis von Fruchtsaft, das heisst Obst oder Gemüsesaft, vor, wie beispielsweise aus der WO-A-8 905 101, DE-A-3 213 744 und der GB-A-2 209 936 bekannt ist. Diese Lebensmittel enthalten eine mehrfach ungesättigte Omega-3-Fettsäure in Form von Fischöl, ein oder mehrere Vitamine, insbesondere Vitamin A, C und E sowie weitere übliche Zusätze wie beispielsweise Emulgatoren, Zucker und Aromastoffe.

Trotz dieser bekannten, vorteilhaften Wirkungen von Fischöl und dessen Inhaltsstoffen ist die Nachfrage nach diesen Produkten ausserordentlich gering, was zweifellos auf den widerwärtigen Geschmack von Fischöl, Fischtran bzw. Lebertran zurückzuführen ist. Um der durch den widerwärtigen Geschmack bedingten Abstinenz entgegenzuwirken, wurde in jüngster Zeit Fischöl in Kapseln in Apotheken angeboten. Dies führt, abgesehen von dem hierdurch bedingten hohen Preis, zu einer Unterdosierung, da der Inhalt einer einzelnen Kapsel zu gering ist, um die gewünschte prophylaktische und/oder heilende Wirkung zu entfalten; die Einnahme einer Vielzahl von derartigen Kapseln jedoch von vielen als unzumutbar empfunden wird, zumal ein nachträgliches unangenehmes Aufstossen nicht auszuschliessen ist.

Der Stand der Technik zeigt, dass es Schwierigkeiten bereitet, Lebensmittel der genannten Art, insbesondere Fruchtsaft-Getränk herzustellen, die nicht nur prophylaktisch und/oder therapeutisch wirksam sind, vom Aussehen, Geschmack und Geruch angenehm sind, eine ausreichende Lagerfähigkeit aufweisen.

Aufgabe der Erfindung ist es daher, ein Verfahren anzugeben, mit dem ein Fruchtsaft-Getränk der eingangs erwähnten Art hergestellt werden kann, das frei von den im Vorhergehenden erwähnten Nachteilen ist und sowohl in geschmacklicher als auch in preislicher Hinsicht annehmbar und attraktiv ist, keinen unangenehmen Geruch aufweist, gesundheitsfördernd und leicht und bequem einzunehmen ist und eine ausreichend hohe Dosierung gewährleistet.

Die gestellte Aufgabe wird gelöst durch
a) das eingangs genannte Verfahren gemäß Anspruch 1; sowie
b) die Anwendungen des Verfahrens gemäß den Ansprüchen 4 bzw. 5.

Das erfindungsgemässe hergestellte Fruchtsaft-Getränk vorzugsweise in Form eines Obst- oder Gemüsesaft-Getränkes zeichnet sich durch angenehmen Geschmack und Geruch aus; es kann somit leicht und ohne Widerwillen konsumiert werden. Der Eigengeschmack der die Komponente A bildenden mehrfach ungesättigten Omega-3-Fettsäure wird durch die übrigen Bestandteile des Getränkes überdeckt oder wird durch die Wechselwirkung zwischen den Komponenten A und B, insbesondere die gegenseitige Stabilisierung, auf die im Folgenden noch näher eingegangen werden wird, unterbunden, so dass er überhaupt nicht in Erscheinung tritt.

Die Komponenten A und B liegen zweckmässigerweise in krankheitsprophylaktischen und/oder krankheitsheilenden Mengen vor. Diese sind variierbar und können gewünschtenfalls auf das Nahrungsangebot in der Region, in der das Fruchtsaft-Getränk zur Konsumation angeboten wird, abgestimmt werden. So kann beispielsweise in einer Region, in der ohnehin viel Fisch verzehrt wird, der Anteil der Komponente A herabgesetzt und umgekehrt in einer Region, in der nur wenig Fisch verzehrt wird, erhöht werden. Ausserdem kann das Vitaminangebot variiert und den Lebensbedingungen angepasst werden, so dass das Auftreten von ortsspezifischen Mangelerscheinungen wirksam verhindert werden kann.

Das Fruchtsaft-Getränk wird in der Regel in abgepackter Form, z.B. in Flaschen, angeboten, wobei vorteilhafterweise jede Packung eine Tagesdosis der Komponenten A und B enthält. Diese kann portionsweise, z.B. in zwei bis vier Portionen, konsumiert werden, wobei die gelegentliche Konsumation grösserer Mengen keinesfalls schädlich wirkt.

Das Fruchtsaft-Getränk enthält als die Komponente A bildende mehrfach ungesättigte Omega-3-Fettsäuren bevorzugt Eicosapentaensäure und/oder Docosahexaensäure oder deren Ester, als Bestandteil von Fischöl Der Gehalt an den genannten Säuren beträgt je nach Fischart und Gegend, in welcher der Fisch gelebt hat, 20 bis bis 33%, bezogen auf die Gesamtmenge des Fettes im Fischöl. Die genannten Säuren können aber auch in reiner Form vorliegen, sofern die reinen Säuren in ausreichenden Mengen und zu einem angemessenen Preis verfügbar sind.

Als Komponente B enthält das Fruchtsaft-Getränk vorzugsweise Vitamin A oder dessen Vorstufe Beta-Carotin, Vitamin C, Vitamin E und/oder Retinoide. Die genannten Vitamine und Provitamine können ganz oder teilweise, insbesondere im Falle eines Obst- oder Gemüsesaft-Getränkes, in dem die Basis bildenden Saft oder Saftgemisch enthalten sein und gegebenenfalls nur ergänzt werden. Um eine ausreichend hohe Dosierung zu erreichen, werden sie dem die Basis bildenden Saft oder Saftgemisch beigemischt. Da es im Falle von Vitamin A durch Ueberdosierung zu unerwünschten und gegebenenfalls schädlichen Nebenwirkungen kommen kann, enthält das Fruchtsaft-Getränk vorzugsweise anstelle von Vitamin A dessen Vorstufe Beta-Carotin, welches vom Körper in den erforderlichen Mengen in Vitamin A übergeführt wird, während der Rest zur Ausscheidung gelangt. Bei den Retinoiden handelt es sich um nahe Verwandte von Vitamin A mit im wesentlichen derselben Wirkung. Die Entwicklung der Retinoide ist noch im Gang mit dem Ziel, Retinoide zu erhalten, die möglichst wenig Nebenwirkungen zeigen.

Die Komponente B ergänzt in wirksamer Weise die Komponente A bei der Prophylaxe von cardiovaskulären Erkrankungen und ist darüberhinaus insbesondere für eine Krebs-Prophylaxe von Bedeutung. Dies umso mehr als die Menge der im Fruchtsaft-Getränk der genannten Art enthaltenen Komponente A für eine wirksame Krebs-Prophylaxe nicht ausreicht, aber nicht ohne weiteres beliebig gesteigert werden kann.

Neben den Komponenten A und B enthält das Fruchtsaft-Getränk vorzugsweise noch Zucker, Süss- und/oder Geschmacks- oder Aromastoffe. Letztere dienen dazu, den den Omega-3-Fettsäuren, die in Form von Fischöl eingesetzt werden, anhaftenden unangenehmen Eigengeschmack bzw. -geruch zu überdecken und dem Lebensmittel einen, auch für empfindliche Gaumen und Nasen angenehmen Geschmack zu verleihen. Ein Gehalt an Geschmacks- und/oder Aromastoffen ist insbesondere dann zu empfehlen, wenn die Geschmacksintensität der mit dem Fischöl kombinierten übrigen Bestandteile zu schwach ist, um den Fischölgeschmack zu überdecken. Die Auswahl geeigneter Geschmacks- oder Aromastoffe liegt im Bereich fachmännischen Könnens und kann anhand von Fachliteratur und Tabellenwerken vorgenommen werden, wobei in der Regel Produkte natürlichen Ursprungs bevorzugt sind.

Ausserdem kann das Fruchtsaft-Getränk noch weitere prophylaktisch oder heilend wirkende Zusätze, z.B. Vitamin D enthalten, um Mangelerscheinungen, die in der Gegend auftreten, in der das Lebensmittel konsumiert werden soll, abzuhelfen.

Das Fruchtsaft-Getränk in Form eines Obst- und/oder Gemüsesaft-Getränkes enthält zweckmässigerweise viskositätserhöhende Zusätze zur Verbesserung der Lagerstabilität, insbesondere zur Verhinderung einer unerwünschten Trennung des als Emulsion vorliegenden Saftes oder Saftgemisches, durch die deren Aussehen beeinträchtigt wird, sowie zur Erhöhung der Oxydationsstabilität. Als viskositätserhöhende Zusätze kommen beispielsweise Fruchtsaftkonzentrate und Pektine in Betracht.

Zur Verbesserung der Lagerstabilität, insbesondere der Stabilität gegenüber oxydierenden Einflüssen ist neben den als Antioxydantien wirkenden Vitaminen ein Gehalt an zusätzlichen Antioxydantien zu empfehlen, vorallem dann, wenn das Lebensmittel in sterilisierter Form angeboten werden soll.

Wie bereits erwähnt, sollte die Packungsgrösse des Fruchtsaft-Getränkes so bemessen sein, dass jede einzelne Packung, z.B. eine Flasche eine Tagesdosis der Komponenten A und B enthält, wobei die Tagesportion beispielsweise 1/3 Liter des Getränkes beträgt.

Bei einem bevorzugten Fruchtsaft-Getränk weist eine Tagesportion von 1/3 Liter die folgende Zusammensetzung auf:

| | |
|---|---|
| Orangensaft | 0,3 Liter |
| Zucker (Sacharose) | 11 g |
| natürliches Orangenaroma | 1,8 g |
| Fischöl | 750 mg |
| Vitamin C | 250 mg |
| Vitamin E | 60 mg |
| Beta-Carotin | 15 mg |
| Wasser zum Auffüllen auf | 1/3 Liter. |

Ein weiteres bevorzugtes Getränk auf der Basis von Obst- und/oder Gemüsesaft enthält in 525 g Fertiggetränk

| | |
|---|---|
| Fruchtsaftbasis (mit ca. 90% Saft) | 472 g |
| Fischöl | 1300 mg |
| Beta-Carotin sowie Süssstoffe, Zucker, Orangenaroma. | 23 mg |

Das erfindungsgemässe Herstellungsverfahren für das Fruchtsaft-Getränk wird nachstehend am Beispiel der Herstellung eines Getränkes auf der Basis eines Obst- und/oder Gemüsesaftes beschrieben. Bei der Durchführung des Verfahrens geht man so vor, dass man
a) die Fettsäuren und/oder deren Ester oder diese enthaltendes Fischöl in Form eines trockenen Pulvers bereitstellt;
b) Vitamin E und eine Teilmenge oder die ganze Menge des Beta-Carotins in Form eines trockenen Pulvers zugibt und diese Mischung mit in trockener Form vorliegendem Vitamin C vermischt;
c) das in Stufe (b) erhalten trockene Gemisch in auf mindestens 40°C erwärmten Obst- und/oder Gemüsesaft einrührt, oder zunächst in auf mindestens 40°C erwärmtes Wasser einrührt und danach dem auf die gleiche Temperatur erwärmten bereitgestellten Obst- und/oder Gemüsesaft hinzufügt, wobei die Menge des Obst- und/oder Gemüsesaftes mehr 60% des Endproduktes beträgt; und
d) das erhaltene Gemisch mit Wasser auf 100% auffüllt, sterilisiert und in Gebinde für den Transport und/oder Verkauf abfüllt,
wobei man in der Verfahrensstufe (c) oder (d) ein Viskositätserhöhendes Mittel, wie beispielsweise Pektine und/oder Fruchtsaftkonzentrat hinzufügt.

Bei diesem Verfahren ist es wesentlich, dass die fettlöslichen Komponenten, in Stufe (a) die Fettsäuren und/oder deren Ester oder diese enthaltendes Fischöl und in Stufe (b) das Vitamin E und das Beta-Carotin, in Form eines trockenen Pulvers eingesetzt werden. Auf diese Weise können aus den unterschiedlichen Lösungsverhalten der einzelnen Komponenten resultierende Nachteile vermieden werden. Bekanntlich sind einzelne der das Getränk bildenden Komponenten wasserlöslich, z.B. Vitamin C, andere nur mit Wasser mischbar und ein Teil fettlöslich. Dieser Umstand führt dazu, dass sich die Komponenten im Getränk nicht ohne weiteres gleichmässig verteilen und die fettlöslich Komponenten beim Stehenlassen des Getränkes in Form von Oelpfützen obenauf schwimmen. Dadurch würde das Aussehen des Getränkes stark beeinträchtigt, was zu einer Ablehnung durch den Konsumenten führen dürfte. Die Verwendung von Lösungsvermittlern und/oder Dispergier- oder Emulgiermitteln führt in der Regel nur zu kurzzeitigen Erfolgen und ist im Zusammenhang mit Lebensmitteln nicht unbedenklich. Der Einsatz der fettlöslichen Komponenten in trockener Form bietet hingegen die Möglichkeit ein Getränk herzustellen, bei dem die genannten Nachteile nicht auftreten und das auch bei länger dauernder Lagerung, z.B. neun bis zwölf Monate, seine Homogenität und sein ansprechendes Aussehen bewahrt. Ausserdem kann auf diese Weise erreicht werden, dass Eigengeruch und Eigengeschmack des Fischöls oder der mehrfach ungesättigten Omega-3-Fettsäuren im fertigen Getränk nicht oder nur wenig spürbar sind und auch ein nachträgliches unangenehmes Aufstossen vermieden wird. Bei der Herstellung des Getränkes wird der Frucht-, d.h. Obst- oder Gemüsesaft in einer Menge verwendet, die mehr als 60% der Gesamtmenge des Endproduktes entspricht. Dem Obst- oder Gemüsesaft werden ausserdem viskositätserhöhende Mittel, beispielsweise vor dem Einrühren des pulverförmigen Materials aus Stufe (b), beigefügt. Die Auswahl der im Einzelfall zu verwendenden viskositätserhöhenden Mittel liegt im Bereich fachmännischen Könnens. In diesem Stadium können auch Zucker, Süss-, Geschmacks- oder Aromastoffe hinzugefügt werden, sofern deren Zugabe erwünscht ist. Die Zugabe von Zucker kann ausserdem eine leichte Erhöhung der Viskosität bewirken, wodurch die Gefahr einer anfänglichen Oxydation der Bestandteile des Getränkes vor der Einstellung des Lösungsgleichgewichtes verhindert werden kann, da mit steigender Viskosität die Beweglichkeit der im Saft oder Saftgemisch enthaltenen Moleküle herabgesetzt wird und dadurch Oxydationsreaktionen sowohl im Herstellungsstadium als auch bei der späteren Lagerung des Getränkes gehemmt werden. Da Verluste durch Oxydation in der Grössenordnung von 10% während der Herstellungs- und Abfüllphase nicht vermieden werden können, empfiehlt es sich, diese Verluste bei der Bemessung der Mengen der einzelnen Komponenten zu berücksichtigen.

Der hohe Saftanteil im fertigen Getränk von mehr als 60%, hat noch einen anderen positiven Aspekt: Er sichert eine bleibende Trübstabilität auch bei relativ hohem Anteil an Fischöl, z.B. 750 mg auf 1/3 Liter entsprechend der im vorhergehenden angegebenen Rezeptur. Versuche haben gezeigt, dass Getränke mit einem kleineren Saftanteil bei gleich hohem Anteil an Fischöl keine befriedigende Dauer-Trübstabilität aufweisen. Es ist anzunehmen, dass es bei geeigneter Abstimmung der Anteile der Komponenten A und B aufeinander zu einer Gleichgewichtseinstellung kommt, wodurch die Trübstabilität auf die Dauer gesichert bleibt. Die Trübstabilität trägt letztlich zu einem ansprechenden Aussehen des Getränkes und damit zu seiner Akzeptanz durch den Konsumenten bei.

Dem gleichen Ziel dient die zusätzliche Zugabe geringer Mengen von Antioxydantien. Die Zugabe der Antioxydantien zum Saft oder Saftgemisch kann gleichzeitig mit der Zugabe der anderen Komponenten oder unmittelbar danach vorgenommen werden, worauf das fertige Gemisch anschliessend oder vor dem Abfüllen sterilisiert werden kann. Ausserdem empfiehlt es sich das Getränk vor dem Abfüllen zu homogenisieren, da auf diese Weise seine Trübstabilität und damit sein Aussehen in günstiger Weise beeinflusst werden kann. Zur Erhöhung der Lagerstabiltät trägt auch bei, wenn das Getränk möglichst bis zum Verbrauch vor der Einwirkung von Licht geschützt wird.

Es war aufgrund der bekannten Eigenschaften der Komponenten A und B keinesfalls zu erwarten, dass deren Kombination und gegenseitige Abstimmung zu einem Getränk führen würde, dessen Eigenschaften hinsichtlich Stabilität (Haltbarkeit) gegenüber oxydierenden Einflüssen, Dauer-Trübstabilität, Lagerstabilität, Vitamingehalt, Aussehen, Geschmack und Geruch gegenüber diesen Eigenschaften der einzelnen Komponenten in einem Mass verbessert sind, das weit über das durch, die Summe der Eigenschaften der Einzelkomponenten gegebene Mass hinausgeht und nur durch ein synergistisches Zusammenwirken erklärbar ist. Die vorteilhafte Verbesserung ist nicht zuletzt darauf zurückzuführen, dass es durch die Kombination der Einzelkomponenten zu einer gegenseitigen Schutzwirkung kommt. So fällt es beispielsweise auf, dass der Vitamingehalt der Kombination, abgesehen von einem anfänglichen geringen Verlust in der Herstellungsphase auch bei länger dauernder Lagerung, z.B. von neun bis zwölf Monaten, unverändert bleibt. Dies war keineswegs zu erwarten, da bekanntlich bei Obst und Gemüse der Vitamingehalt aufgrund von Abbaureaktionen in der Regel bereits kurz nach der Ernte rapide abnimmt und bereits nach verhältnismässig kurzer Zeit gegen null geht. Es kann angenommen werden, dass sich die einzelnen Komponenten gegenseitig stabilisieren, wobei offenbar Beta-Carotin und Vitamin C eine besondere Bedeutung zukommt.

Weit bedeutungsvoller und besonders hervorzuheben ist jedoch der Umstand, dass sich die physiologischen Wirkungen der Komponenten A und B in synergistischer Weise ergänzen, so dass der durch das Fruchtsaft-Getränk zu erzielende prophylaktische und/oder heilende Effekt wesentlich grösser ist, als aufgrund der den Einzelkomponenten eigenen Wirkungen zu erwarten war. Darüberhinaus bewirkt der Kombinationseffekt eine ausserordentliche Verbreiterung des Wirkungs- und Anwendungspektrums, insbesondere im Zusammenhang mit der Anwendung im Bereich der Prophylaxe und Heilbehandlung bei cardiovaskulären Erkrankungen sowie im Bereich der Krebsprophylaxe und der Krebstherapie. Ein breites Wirkungs- und Anwendungsspektrum ist aber aufgrund der Komplexität der genannten Erkrankungen, die eine Vielzahl von örtlich und pathologisch unterschiedlichen Krankheiten umfassen, eine massgebliche Voraussetzung für eine erfolgreiche Prophylaxe und/oder Therapie. Gerade im Fall von Krebs darf nicht übersehen werden, dass sich die verschiedenen Arten von Krebs nicht nur durch den Ort ihres Auftretens sondern auch durch Genese und Pathologie unterscheiden. Eine wirksame Krebsprophylaxe kann deshalb nur durch ein Zusammenwirken mehrerer Komponenten erreicht werden, die sich ergänzen und gegenseitig potenzieren, wie dies bei dem erfindungsgemässen Fruchtsaft-Getränk aufgrund des beobachteten und zweifelsfrei vorhandenen Synergismus der Fall ist. Bei der Einnahme eines einzelnen Vitamins oder einer ungesättigten Omega-3-Fettsäure wird in der Regel nur eine sehr begrenzte Wirkung erreicht, derart, dass die einzelne Wirkstoffkomponente für sich allein ihre Wirkung nur in einem sehr engen Bereich, beispielsweise nur bei einem spezifischen Krankheitsbild, entfaltet, bei einem anderen, ähnlichen Krankheitsbild aber kaum oder gar nicht wirksam ist. Dies wirkt sich insbesondere im Falle der Krebsprophylaxe und der Krebstherapie nachteilig aus, da gerade bei dieser Krankheit erhebliche Unterschiede hinsichtlich der Art, des Ortes des Auftretens, der Genese und der Pathologie bestehen. Es war deshalb ausserordentlich überraschend und keineswegs voraussehbar, dass durch die Kombination der Komponenten A und B sowie durch die Auswahl und Kombination der diese Komponenten bildenden Ingredientien ein prophylaktisch und/oder heilend wirkendes Fruchtsaft-Getränk mit einem derart breiten Anwendungs- und Wirkungsspektrum geschaffen werden könnte.

Auf diese Weise wird in einmaliger Weise die Möglichkeit erschlossen, dem Körper die für eine wirksame Prophylaxe notwendigen Wirkstoffe, nämlich ungesättigte Omega-3-Fettsäuren einerseits und Vitamine andererseits, in ausreichenden Mengen und auf einfache und angenehme Weise zuzuführen, ohne die Gefahr unerwünschter Nebenwirkungen heraufzubeschwören

## Patentansprüche

1. Verfahren zur Herstellung eines Fruchtsaft-Getränkes mit einem Gehalt an:
A) Mindestens einer prophylaktisch und/oder heilend wirkenden mehrfach ungesättigten Omega-3-Fettsäure und/oder deren Ester; und
B) einen oder mehreren Vitaminen und/oder Provitaminen mit der gleichen oder einer weiteren prophylaktischen oder heilenden Wirkung,
dadurch gekennzeichnet, dass man
a) als Komponent A Fischöl in Form eines trockenen Pulvers bereitstellt;
b) als Komponente B Vitamin E und eine Teilmenge oder die ganze Menge eines Beta-Carotins in Form eines trockenen Pulvers zugibt und diese Mischung mit in trockener Form vorliegendem Vitamin C vermischt;
c) das in Stufe (b) erhaltene trockene Gemisch in auf mindestens 40° C erwärmten Fruchtsaft einrührt, oder zunächst in auf mindestens 40°C erwärmtes Wasser einrührt und danach dem auf die gleiche Temperatur erwärmten bereitgestellten Fruchtsaft hinzufügt, wobei die Menge des Fruchtsaftes mehr als 60 % des Endproduktes beträgt; und
d) das erhaltene Gemisch mit Wasser auf 100 % auffüllt und sterilisiert und dann in Gebinde abfüllt,
wobei man in der Verfahrensstufe (c) oder (d) ein viskositätserhöhendes Mittel, wie beispielsweise Pektine, hinzufügt.

2. Verfahren nach Anspruch 1, dadurch gekennzeichnet, dass man in Verfahrensstufe (b) oder (c) Zucker, Süss-, Geschmacks- oder Aromastoffe zugibt.

3. Verfahren nach Anspruch 1 oder 2, dadurch gekennzeichnet, dass man das erhaltene Gemisch homogenisiert.

4. Anwendung des Verfahrens nach einem der Ansprüche 1 bis 3 zur Herstellung eines Orangensaft-Getränkes der folgenden Zusammensetzung:
| | |
|---|---|
| Orangensaft | 0,3 Liter |
| Zucker (Sacharose) | 11 g |
| natürliches Orangenaroma | 1.8 g |
| Fischöl | 750 mg |
| Vitamin C | 250 mg |
| Vitamin E | 60 mg |
| Beta-Carotin | 15 mg |
| Wasser zum Auffüllen auf | 1/3 Liter. |

5. Anwendung des Verfahrens nach einem der Ansprüche 1 bis 3 zur Herstellung eines Fruchtsaft-Getränkes enthaltend in 525 g Fertiggetränk:
| | |
|---|---|
| Fruchsaftbasis (mit ca. 90 % Saft) | 472 g |
| Fischöl | 1300 mg |
| Beta Carotin | 23 mg |
| sowie Süsstoffe, Zucker, Orangenaroma. | |

## Claims

1. A process for the production of a beverage on the basis of fruit and/or vegetable juice, with a content of:
A) at least one multiply unsaturated omega-3-fatty acid and/or its esters with a prophylactic and/or healing effect; and
B) one or several vitamins and/or provitamins with the same or a further prophylactic or healing effect,
characterised in that
a) component A is provided as fish oil in the form of a dry powder;
b) component B is added as vitamin E and a partial amount or the entire amount of a beta carotene, in the form of a dry powder, and this mixture is mixed with vitamin C available in dry form;
c) the dry mixture obtained in step (b) is stirred into fruit and/or vegetable juice heated to at least 40° C or, to begin with, stirred into water heated to at least 40° C and thereafter added to the fruit and/or vegetable juice heated to the same temperature, the quantity of the fruit and/or vegetable juice amounting to more than 60% of the final product; and
d) the resulting mixture is brought up to 100% using water, sterilized and then filled into containers,
where at process stage (c) or (d) a viscosity increasing agent, such as pectin, is added.

2. Process according to claim 1, characterised in that sugar, sweetener, flavoring or aromatic substances are added at process stage (b) or (c).

3. Process according to claim 1 or 2, characterised in that the resulting mixture is homogenised.

4. Application of the process according to one of claims 1 to 3 for the production of a beverage on the basis of orange juice, having the following composition:
| | |
|---|---|
| orange juice | 0.3 liter |
| sugar (saccharose) | 11 g |
| natural orange aroma | 1,8 g |
| fish oil | 750 mg |
| vitamin C | 250 mg |
| vitamin E | 60 mg |
| beta carotene | 15 mg |
| water to give | 1/3 liter. |

5. Application of the process according to one of claims 1 to 3 for the production of a beverage on the basis of fruit and/or vegetable juice containing in 525 mg of ready-to-use beverage:
| | |
|---|---|
| fruit juice base (with about 90% juice) | 472 g |
| fish oil | 1300 mg |
| beta carotene | 23 mg |
| as well as sweeteners, sugar, orange aroma. | |

## Revendications

1. Procédé de production d'une boisson à base de jus de fruits et légumes, avec une teneur de;
A) au moins un acide gras omega-3 poly-insaturé et/ou ses esters, ayant un effet prophylactique et/ou guérissant; et
B) une ou plusieures vitamines et/ou provitamines ayant le même et/ou un autre effet prophylactique et/ou guérissant,
caractérisé en ce que
a) l'on met à disposition en tant que composante A de l' huile de poisson sous forme de poudre sèche;
b) l'on ajoute en tant que composante B de la vitamine E ainsi qu'une quantité partielle ou bien la quantité entière d'un beta-carotène sous forme de poudre sèche et l'on mélange cette mixture à de la vitamine C présente sous forme sèche;
c) l'on introduit sous agitation le mélange sec obtenu à l'étape (b) dans du jus de fruits et légumes chauffé au moins à 40° C ou bien on l'introduit d'abord sous agitation dans de l'eau chauffée au moins à 40° C et ensuite on l'ajoute au jus de fruits et légumes mis à disposition et chauffé à la même température, la quantité du jus de fruits et légumes constituant plus de 60% du produit final; et
d) l'on amène à 100% avec de l'eau le mélange ainsi obtenu, on le stérilise et ensuite on le met en récipients,
un agent pour élever la viscosité, comme par example des pectines, étant ajouté au cours de l'étape (c) ou (d) du procédé.

2. Procédé selon la revendication 1, caracterisé en ce que du sucre, des substances édulcorantes, des substances pour améliorer le goût, ou des substances aromatisantes sont ajoutées au cours de l'étape (b) ou (c) du procédé.

3. Procédé selon l'une des revendications 1 ou 2, caractérisé en ce que le mélange résultant est homogénéisé.

4. Utilisation du procédé selon l'une des revendications 1 à 3 dans la production d'une boisson à base de jus d'orange avec la composition suivante:
| | |
|---|---|
| jus d'orange | 0,3 litre |
| sucre (saccharose) | 11 g |
| arôme naturel d'oranges | 1.8 g |
| huile de poisson | 750 mg |
| vitamine C | 250 mg |
| vitamine E | 60 mg |
| beta-carotène | 15 mg |
| eau pour amener à | 1/3 litre. |

5. Utilisation du procédé selon l'une des revendications 1 à 3 dans la production d'une boisson de jus de fruits et légumes contenant dans 525 mg de boisson prête à la consommation:
| | |
|---|---|
| base de jus de fruits et légumes (avec environ 90% de jus) | 472 g |
| huile de poisson | 1300 mg |
| beta-carotène | 23 mg |
| ainsi que édulcorants, sucre, arôme d'oranges. | |
